# FASCICULE DE BREVET EUROPEEN

(11) **EP 2 349 225 B1**
(45) Date de publication et mention de la délivrance du brevet: **06.12.2017**
(21) Numéro de dépôt: 09756035.3
(22) Date de dépôt: 15.10.2009
(51) Int. Cl.: A61K 8/11, A61K 8/892, A61Q 13/00, A61Q 17/04, A61Q 19/00, B01J 13/16, B01J 13/18

(54) **MICROCAPSULES AYANT UNE ENVELOPPE COMPOSÉE ESSENTIELLEMENT D'HOMOPOLYMÈRES OU DE COPOLYMÈRES SILSESQUIOXANE**
MIKROKAPSELN MIT EINER IM WESENTLICHEN AUS SILSESQUIOXAN-HOMOPOLYMEREN ODER COPOLYMEREN ZUSAMMENGESETZTEN HÜLLE
MICROCAPSULES HAVING AN ENVELOPE COMPOSED ESSENTIALLY OF SILSESQUIOXANE HOMOPOLYMERS OR COPOLYMERS

(30) Priorité: 20.10.2008 FR 0857111
(43) Date de publication de la demande: 03.08.2011
(73) Titulaire: Microcapsules Technologies, 45390 Puiseaux (FR)
(72) Inventeur: HABAR, Gérard, F-77780 Bourron-Marlotte (FR)
(74) Mandataire: Le Cloirec, Claudine
(86) Numéro de dépôt international: PCT/FR2009/051970
(87) Numéro de publication internationale: WO 2010/046583

(56) Documents cités:
- EP-A- 0 216 388
- EP-A- 0 661 334
- EP-A- 1 426 100
- US-A- 3 257 330
- US-A- 3 551 346
- US-B1- 6 251 313
- AHN BOK YEOP ET AL: "Core/shell silica-based in-situ microencapsulation: a self-templating method." CHEMICAL COMMUNICATIONS (CAMBRIDGE, ENGLAND) 14 JAN 2006, no. 2, 14 janvier 2006 (2006-01-14), pages 189-190, XP009120297 ISSN: 1359-7345
- SEOK, S ET AL: "Microencapsulation of oil in organically modified silicate glass by sol-gel process" MATERIALS RESEARCH SOCIETY SYMPOSIUM PROCEEDINGS, VOL. 726 (ORGANIC/INORGANIC HYBRID MATERIALS ), 2002, pages 193-198, XP009120303 ISSN: 0272-9172

## Description

La présente divulgation se rapporte à des microcapsules de type core shell ou à réservoir comportant chacune un noyau (généralement liquide) entouré par une enveloppe (généralement solide) composée essentiellement d'homopolymères ou de copolymères silsesquioxane, ainsi qu'à leur utilisation pour fabriquer des produits cosmétiques.

La présente invention se rapporte au procédé de fabrication des microcapsules susmentionnées, tel que revendiqué dans la revendication 1. Les microcapsules renfermant un principe actif lipophile ou hydrophile sont utilisées dans de nombreux domaines, par exemple, dans les domaines des cosmétiques ou de la pharmacie. Des principes actifs, tels que du parfum, des filtres UV ou encore des médicaments peuvent être insérés dans des microcapsules afin d'y être protégés, puis lentement libérés.

Il existe deux types de microcapsules en fonction du caractère hydrophile ou lipophile du principe actif contenu dans les microcapsules. Ainsi, lorsque les microcapsules contiennent une phase interne aqueuse, la phase continue est organique et lorsqu'elles contiennent une phase interne organique, la phase continue est aqueuse.

De nombreuses microcapsules ont été développées dans l'art antérieur, notamment des microcapsules à base de silsesquioxane, qui est un composé bon marché, facilement disponible et présentant de nombreux avantages. Il présente une bonne stabilité thermique et mécanique, il est résistant à la lumière et est biologiquement inactif. Il est par conséquent bien toléré par la peau, notamment la peau humaine.

De façon connue, les silsesquioxanes se réfèrent à la formule générale empirique R-SiO_{3/2} où Si est l'élément silicium, O est l'oxygène et R est un groupe alkyle, alcène, aryle, arylène. Les silsesquioxanes sont obtenus généralement par hydrolyse et condensation d'organotrialcoxysilanes répondant à la formule empirique générale : .R-Si(OR₁)₃ où R est tel que défini précédemment, et R₁ est un radical généralement alkyle.

Le document US 3 257 330 décrit notamment un procédé de fabrication d'une particule à base de gel coloré comprenant un organopolysiloxane en tant que matrice. Toutefois, lorsqu'un alcoxysilane présentant un groupe organique hydrophobe, tel que le méthyltriéthoxysilane, est utilisé en tant que matériau de départ de la matrice (réaction d'hydrolyse), la composition polymère forme alors dans une solution aqueuse, un dépôt.

Par conséquent, il est difficile de fabriquer une microcapsule en incorporant un noyau hydrophobe lors de la polymérisation d'un hydrolysat d'un alcoxysilane dans une solution aqueuse.

Le document US 3 551 346 décrit dans son art antérieur, un procédé de fabrication de microcapsules dans lequel un polysiloxane est synthétisé à partir d'un trialcoxysilane. Toutefois, l'enveloppe des microcapsules composée du polysiloxane ne présente pas une résistance et une dureté suffisantes pour convenir à l'encapsulation de principes actifs. C'est pourquoi, la solution trouvée par ce document est de fabriquer des microcapsules comprenant une paroi à deux couches.

Tel qu'il est indiqué dans ce document, il est difficile à l'heure actuelle de fabriquer une microcapsule ayant une seule enveloppe à base d'organopolysiloxane.

Le brevet US 6 251 313 décrit des microcapsules ayant une paroi d'organopolysiloxane fabriquée par polymérisation en milieu basique en présence de monomères silane aminés.

L'inconvénient d'un tel procédé réalisé en milieu basique est non seulement la présence d'une porosité résiduelle dans la paroi d'organopolysiloxane, mais également un jaunissement des microcapsules à la lumière engendré par les groupes amines présents. De plus, l'inconvénient de cette technique en milieu basique est que le polymère en formation possède d'emblée une structure tridimensionnelle qui se fige rapidement et conduit inévitablement à des microcapsules poreuses.

Ainsi, les solutions trouvées par l'état de la technique sont de faire intervenir : soit plusieurs monomères spécifiquement dosés rendant la réaction compliquée et onéreuse ; soit des copolymères difficilement synthétisables portant de longues chaînes afin d'assouplir la structure, toutefois dans ce cas les copolymères réagissent lentement ; ou soit de diminuer la fonctionnalité des monomères, cependant dans ce dernier cas la réactivité est diminuée et la structure finale fragilisée.

Le document EP 0 661 334 décrit de fines particules de gomme de silicone d'un diamètre moyen de 0,1µm à 100µm comprenant un revêtement à base de résine de polyorganosilsesquioxane, ce revêtement représentent 1 à 500 parties en poids pour 100 parties en poids de particules de gomme de silicone. Ce document décrit une technique de greffage sur des particules solides. En effet, les particules solides de gomme de silicone (cured silicone rubber) sont recouvertes d'une résine de polyorganosilsesquioxane en faisant régir (réaction d'hydrolyse et de condensation) un composé trialcoxysilane avec une dispersion aqueuse de gomme de silicone. En outre, avec le procédé tel que décrit dans ce document, il n'est pas possible d'obtenir des microcapsules majoritairement à base de silsesquioxane car selon ce procédé, les alcoxysilanes polymériseraient non pas autour des gouttelettes de principes actifs liquides mais se polymériseraient sous forme de petite particules dans la phase aqueuse.

Le document EP 1 426 100 décrit des particules formées d'un polymère de type silsesquioxane, comme le phénylpropylsilsesquioxane de l'exemple 1, au sein duquel est absorbé un principe actif (colorant capillaire, filtres UVA, UVB, flavonoïdes...). Ce document ne décrit donc pas des microcapsules à réservoir présentant un noyau (phase lipophile ou phase aqueuse) entouré par une enveloppe externe (polymère).

La publication « core/shell silica-based in situ microencapsultaion : e self-templating method » de Bok Yeop Ahn décrit des microcapsules comprenant un noyau actif lipophile (liquide) et un revêtement solide constitué de silice (SiO₂) et de (RSiO_{1,5})₁₋ₓ-(SiO₂)ₓ, R étant une groupe alkyl et x allant de 0,1 à 0,5. La silice est formée à partir de tétraéthoxysilane (TEOS) et le deuxième composé (RSiO_{1,5})₁₋ₓ-(SiO₂)ₓ est lui-même formé par une combinaison de Si(OR)4 et de RSi(OR')₃ comme le méthyltriméthoxysilane (MTMS). Par conséquent dans ce document le composé silsesquioxane n'est qu'un additif venant en complément du prépolymère de silice. D'ailleurs, dans l'exemple, il ne représente pas plus de 30% du revêtement.

De même la publication « microencapsulation of oil in organically modified silicate glass by sol-gel process » de Sang I. Seok décrit un procédé de préparation de microcapsule comprenant un noyau lipophile (le xylène) et une enveloppe à base principalement de silice et d'un composé de type silsesquioxane en tant qu'additif. La première étape du procédé de ce document consiste à :
- hydrolyser et à condenser du tétraéthyloryhosilicate et du méthyltriméthoxysilane (MTMS) avec de l'eau désionisée, de manière à former un composé oligomère,
- d'éliminer simultanément l'alcool formé lors de l'hydrolyse
- de mélanger, après refroidissement, le composé oligomère obtenu avec un composé lipophile : xylène (phase huileuse) avec un dopant, et
- et d'homogénéiser le mélange phase huileuse/oligomère de manière à former une microémulsion eau dans l'huile.

Le document EP 0 216 388 concerne un procédé pour enlever des polluants atmosphériques (NOₓ, SO₂) à partir d'un gaz.

Aucun de ces documents ne décrit une microcapsule à réservoir dont le revêtement serait essentiellement à base d'un composé de type silsesquioxane.

### Résumé de l'invention :

La présente invention a pour but de proposer un nouveau procédé de fabrication de microcapsules renfermant un principe actif lipophile ou hydrophile qui évitent tout ou en partie aux inconvénients précités.

La présente invention a pour objet un nouveau procédé de fabrication de microcapsules à réservoir comprenant un noyau comprenant au moins un principe actif, ledit noyau étant entouré par une enveloppe polymère formée de 50 à 100 % en poids d'un composé de type silsesquioxane, par rapport au poids total de ladite enveloppe, ledit procédé comprenant les étapes consistant à :
(i) disperser au moins un principe actif lipophile ou hydrophile dans une phase continue respectivement aqueuse ou organique, de manière à former une émulsion ou une dispersion huile dans l'eau ou eau dans l'huile, respectivement,
(ii) hydrolyser un précurseur du composé polymère de type silsesquioxane de formule R- SiO_{3/2} où R est :
   - un radical alkyle à 1 à 20 atomes de carbone, substitué ou non tel que des radicaux méthyle, éthyle, n-propyle, isopropyle, 1-n-butyle, 2-n-butyle, isobutyle, tert-butyle, n-pentyle, isopentyle, néopentyle, tert-pentyle, hexyle, heptyle, octyle, 2,2,4-triméthylpentyle, nonyle, décyle, dodécyle, octadécyle, cycloalkyle, aryle, alkaryle, et aralkyle,
   - un radical alkyle oxygéné, tel les méthoxyéthyle et éthoxyéthyle,
   - un radical halogéné tel que le chloropropyle, 3,3,3-trifluoro-n- propyle, 2,2,2,2,2',2',2'-hexalluoroisopropyle, heptafluoroisopropyle, o-, m- et p-chlorophényle,
   - ou un radical insaturé tel que vinyle, 5-hexényle, 2,4-divinylcyclohexyléthyle, 2-propényle, allyle, 3-butényle, 4-pentényle, éthynyle, propargyle et 2-propynyle.
et le polymériser *in situ* dans ou en contact de la phase aqueuse de la dispersion ou de l'émulsion huile dans l'eau ou eau dans l'huile, de manière à former un homopolymère ou copolymère silsesquioxane
caractérisé en ce (iii) qu'on introduit dans la phase organique des microcapsules en début de réaction d'hydrolyse et/ou de polymérisation, un composé choisi parmi :
- un silicate préférentiellement insoluble dans l'eau à l'état hydrolysé, tel que du polysilicate d'éthyle,
- un précurseur du composé polymère de type silsesquioxane,
- ou leurs mélanges,
et en ce que l'étape de polymérisation s'effectue en milieu acide,
de sorte à conférer à la polymérisation ou à l'encapsulation un caractère interfacial favorable à l'étanchéité des microcapsules.

Une microcapsule à réservoir (ou core shell) comporte un noyau entouré par une enveloppe en polymère. Généralement, le noyau est plus ou moins liquide et le revêtement plus ou moins solide. Ainsi, la microcapsule, comme son nom l'indique, est composée d'une capsule formée par une enveloppe continue en polymère de silsesquioxane entourant un noyau composé lui-même de principes actifs. Le but d'une microcapsule à réservoir est de contenir à l'intérieur de l'enveloppe polymère, qui doit être solide et résistante, un noyau formé de principes actifs. Ce type de technologie est différent de la technique de greffage ou le revêtement est greffé à des particules solides (exemples gomme de silicone) ou la technique de type matrice où des principes actifs sont absorbés sur un polymère solide, il n'y a pas d'enveloppe extérieur (le polymère est synthétisé, puis l'actif y est incorporé).

Comme les microcapsules à réservoir préparées selon le procédé de la présente invention comportent une paroi essentiellement à base d'un composé de type silsesquioxane, elles présentent une résistance et une étanchéité suffisantes pour convenir à l'encapsulation de principes actifs lipophiles ou hydrophiles.

De préférence, le composé polymère de type silsesquioxane représente 70 % ou plus, en poids, par rapport au poids total de ladite enveloppe.

Avantageusement, le composé polymère de type silsesquioxane est R-SiO_{3/2} où R est un radical alkyle à 1 à 20 atomes de carbone, substitué ou non tel que des radicaux méthyle, éthyle, n-propyle, isopropyle, 1-n-butyle, 2-n-butyle, isobutyle, tert-butyle, n-pentyle, isopentyle, néopentyle, tert-pentyle, hexyle tel que n-hexyle, heptyle tel que n-heptyle, octyle tel que n-octyle ou isooctyle, 2,2,4-triméthylpentyle, nonyle, décyle, dodécyle, octadécyle, cycloalkyle tel que cyclopentyle, cyclohexyle et cycloheptyle et méthylcyclohexyle, aryle tel que des radicaux phényle, naphtyle, anthryle et phénanthryle, alkaryle tel que o-, m- et p-tolyle, xylyle et éthylphényle, et aralkyle tels que benzyle, alpha- et béta-phényléthyle.

Préférentiellement, le ou les principes actifs sont choisis parmi: les acides et alcools gras, les solvants organiques, les hydrocarbures, les esters, les fluides et gommes silicone, les huiles végétales et extraits végétaux lipophiles ou hydrophiles, les colorants réactifs ou non ainsi que les dispersions de pigments, les filtres UV, les vitamines et molécules médicalement actives pures ou en solution aqueuse ou organique, les parfums et arômes, les insecticides et répulsifs, les catalyseurs, les matériaux à changement de phase, les composés phénoliques, l'eau, les désinfectants tels que l'eau oxygénée, le glutaraldéhyde en solution, les sels, les aminoacides, les protéines, les polypeptides, les enzymes, la DHA, les saccharides et les polysaccharides, les sels d'amine ou leurs mélanges.

L'ajout d'un silicate préférentiellement insoluble ou d'un précurseur du composé polymère silsesquioxane dans la phase organique du mélange permet en effet d'obtenir des microcapsules à base essentiellement de silsesquioxane.

Les études de la société demanderesse ont montré en effet de façon surprenante et inattendue, que si lors de la fabrication de microcapsules, le polymère ou copolymère silsesquioxane est synthétisé *in situ* par hydrolyse et polymérisation en milieu acide, alors il était possible d'obtenir plus aisément (par rapport à un milieu basique) des microcapsules ayant une seule enveloppe composée de polymère de type silsesquioxane résistante et étanche.

En conséquence, compte tenu des inconvénients existants pour fabriquer des microcapsules à base de silsesquioxane, un homme du métier n'aurait pas été enclin à réaliser les étapes d'hydrolyse et de polymérisation en milieu acide. En effet, dans les techniques décrites précédemment, le durcissement s'effectue toujours par augmentation du pH, en le portant dans le domaine basique où les réticulations de polymérisation sont rapides et complètes.

De préférence, le pH lors de la polymérisation est inférieur à 6.

Selon une première variante de réalisation, le pH est situé entre 3 et 5 lors de l'hydrolyse et lors du début de la polymérisation, puis de 1 à 4, de préférence de 1,5 à 2, 5 jusqu'à la fin de la polymérisation.

Selon une seconde variante de réalisation, le pH se situe entre 1 et 4 dès l'étape d'hydrolyse.

Avantageusement, des ions fluorures ou un ou plusieurs composés comportant dans leur structure des ions fluorures sont présents dans le milieu au cours de la polymérisation.

En particulier, les ions fluorures sont utilisés en présence d'un composé portant une fonction amine.

De manière avantageuse, le pH en fin de réaction de polymérisation est remonté entre 5,5 et 8,5, de préférence entre 6 et 7.

Préférentiellement, dans le cas d'une émulsion huile dans eau, un ou des silanes porteurs de groupes hydrophiles sont introduits après solidification au moins partielle de la paroi des microcapsules.

Avantageusement, dans le cas d'une émulsion eau dans l'huile, un ou des silanes porteurs de groupes lipophiles sont introduits après solidification au moins partielle de la paroi des microcapsules.

Selon les deux caractéristiques ci-dessus, éventuellement au moins un silane porte des charges cationiques.

De manière avantageuse, la température se situe entre 10°C et 50°C lors de l'étape de dispersion ou d'hydrolyse, puis de 40°C à 90°C lors de l'étape de polymérisation.

De préférence, le précurseur du composé polymère de type silsesquioxane est du type R-Si(R₁R₂R₃), où R est tel que défini précédemment,
où R₁,R₂,R₃ désignent chacun indépendamment un groupe : acétoxy, amino, acide amide, oximino, chlore, un groupe OR₄ où R₄ est :
- un radical alkyle à 1 à 3 atomes de carbone, substitué ou non tels par exemple par des radicaux méthyle, éthyle, n-propyle, isopropyle,
- un radical alkyle oxygéné, tel que les méthoxyéthyle et éthoxyéthyle,
- ou un radical insaturé tel que vinyle, 2-propényle, allyle.

En particulier, le précurseur du composé polymère de type silsesquioxane est le méthyltriméthoxysilane (MTMS), le méthyltriéthoxysilane (MTES), le méthyltrichlorosilane ou leurs mélanges.

La microcapsule à réservoir telle que préparée par le procédé selon l'invention peut être utilisée, pour fabriquer un produit cosmétique ou pharmaceutique présentant un filtreUV.

### Description détaillée de l'invention :

Les préparations d'encapsulation huile dans l'eau, puis eau dans l'huile vont être présentées ci-dessous, suivies d'exemples non limitatifs.

### a) Encapsulation huile dans l'eau :

Dans le cas d'une encapsulation huile dans l'eau, une phase interne lipophile (principes actifs lipophiles) est dispersée dans une phase continue aqueuse.

### Préparation de la phase interne lipophile :

Afin de préparer une phase interne lipophile, un ou plusieurs actifs lipophiles sont mélangés.

Les principes actifs qui comprennent également les substances grasses sont choisis par exemple parmi les: agents antioxydants, antiradicaux libres, mélanorégulateurs, accélérateurs de bronzage, dépigmentants, de coloration de la peau, liporégulateurs, amincissants, anti-acnéiques, antiséborrhéiques, antivieillissement, antirides, anti-UV, kératolytiques, anti-inflammatoires, rafraîchissants, cicatrisants, protecteurs vasculaires, antibactériens, antifongiques, antitranspirants, déodorants, conditionneurs des cheveux, immunomodulateurs, nourrissants, les huiles essentielles et les parfums.

Plus particulièrement, on peut citer comme exemples d'actifs lipophiles pour le traitement de la peau et/ou des cheveux, utilisables dans le cadre de la présente invention les composés suivants : D-alpha-tocophérol, DL alpha tocophérol, acétate de D alpha tocophérol, acétate de DL alpha tocophérol, palmitate d'ascorbyle, glycérides de vitamine F, vitamines D et notamment vitamine D2 et vitamine D3, rétinol, esters de rétinol (palmitate de rétinol, propionate de rétinol), beta carotène, D panthénol, farnésol, acétate de farnésyle, huiles riches en acides gras essentiels et notamment huiles de jojoba et de cassis, acide n octanoyl-5 salicylique, acide salicylique, alkylesters d'alpha-hydroxyacides tels que acide citrique, acide lactique et acide glycolique, acide asiatique, acide madécassique, asiaticoside, extrait total de Centella Asiatica, acide beta glycyrrhétinique, alpha bisabolol, céramides et notamment le 2 oléoylamino-1,3 octadécane, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées (Algoxan red commercialisé par Algatec), huile essentielle de bergamote, octylmethoxycinnamate (Parsol MCX commercialisé par givaudan-Roure), butylméthoxydibenzoylméthane (Parsol 1789 commercialisé par givaudan-Roure), octyl triazone (Uvinul T150 commercialisé par BASF), oxydes de fer jaune, brun, noir, rouge, oxydes de titane, pouvant se présenter sous forme micrométrique ou nanométrique ou sous forme enrobée (par exemple par un perfluoroalkyl), di-tertiobutyl-3,5 hydroxy-4 benzylidène-3 camphre, 2-benzotriazol -2-yl-4-méthyl-6-[3-[1,3,3,3-tétraméthyl-1-[(triméthylsilyl)oxy]-disiloxanyl]-2-méthyl propyl]phénol, huile perfluorée (perfluorodécaline, perfluorooctyl bromure), huile de maïs hyperoxygénée (Epaline 100 commercialisée par la société Carilene).

A ce mélange, dans une variante de réalisation, il est possible d'ajouter à ce mélange de principes actifs lipophiles, un précurseur du composé polymère de type silsesquioxane.

De préférence, le précurseur du composé polymère de type silsesquioxane est du type R-Si(R₁R₂R₃) dans lequel R représente un radical non hydrolysable, et R₁, R₂, R₃ des radicaux hydrolysables.

R est en particulier :
- un radical alkyle à 1 à 20 atomes de carbone, substitué ou non tels par exemple par des radicaux méthyle, éthyle, n-propyle, isopropyle, 1-n-butyle, 2-n-butyle, isobutyle, tert-butyle, n-pentyle, isopentyle, néopentyle, tert-pentyle, hexyle tel que n-hexyle, heptyle tel que n-heptyle, octyle tel que n-octyle ou isooctyle, 2,2,4-triméthylpentyle, nonyle, décyle, dodécyle, octadécyle, cycloalkyle tel que cyclopentyle, cyclohexyle et cycloheptyle et méthylcyclohexyle, aryle tel que des radicaux phényle, naphtyle, anthryle et phénanthryle, alkaryle tel que o-, m- et p-tolyle, xylyle et éthylphényle, et aralkyle tels que benzyle, alpha- et béta-phényléthyle,
- un radical alkyle oxygéné, tel les méthoxyéthyle et éthoxyéthyle,
- un radical halogéné tel que le chloropropyle, 3,3,3-trifluoro-n-propyle, 2,2,2,2',2',2'-hexafluoroisopropyle, heptafluoroisopropyle, o-, m- et p-chlorophényle,
- ou un radical insaturé tel que vinyle, 5-hexényle, 2,4-divinylcyclohexyléthyle, 2-propényle, allyle, 3-butényle et 4-pentényle, éthynyle, propargyle et 2-propynyle ;
et R₁, R₂, R₃ désignent des groupes hydrolysables tels que méthoxy, éthoxy, propoxy et isopropoxy, méthoxyethoxy, acétoxy, amino, acide amide, oximino, des atomes de chlore.

Les réactions d'hydrolyse conduisent au monomère R-Si (OH)₃.

Seront préférées les chaînes courtes qui donnent des vitesses de réaction plus élevées.

Les précurseurs présentant des chaînes courtes seront utilisés de préférence car ils donnent des vitesses de réaction plus élevées.

De préférence, le précurseur du composé polymère de type silsesquioxane est le méthyltriméthoxysilane (MTMS), le méthyltriéthoxysilane (MTES), le méthyltrichlorosilane, ou leurs mélanges. L'intérêt de ces composés est qu'ils conduisent rapidement dans des conditions appropriées à des microcapsules possédant une paroi dure, très résistante chimiquement et microbiologiquement et très peu poreuse.

Il est également possible d'ajouter à ce mélange de principes actifs lipophiles, un organosilicate restant préférentiellement insoluble dans l'eau à l'état hydrolysé, tel que du polysilicate d'éthyle. Dans une variante de réalisation, ce composé peut être introduit également en début de réaction d'hydrolyse et/ou de polymérisation. Cette technique permet de mieux "ancrer" le silsesquioxane en formation à la microcapsule et de diminuer également l'hydrophilie de l'ensemble.

Ces composés : le polysilicate d'éthyle ou le précurseur du composé polymère de type silsesquioxane (MTMS, MTES), lorsqu'ils sont ajoutés à la phase lipophile, permettent de manière surprenante de donner à la polymérisation un caractère partiellement interfacial.

Selon l'art antérieur, dans une encapsulation *in situ,* la polymérisation se fait dans la phase aqueuse. Lors de la polymérisation de monomères de type organosilane et ce, afin de former un polymère de type silsesquioxane (ou autre silicone), il y a formation de R-Si(OH)₃, puis polymérisation avec formation d'un polymère qui contient beaucoup de groupes OH. Au fur et à mesure que la réaction se poursuit, le nombre de groupes OH diminue. Ce polymère en formation est donc au départ très hydrophile, il n'a donc pas tendance tout de suite à se déposer autour des gouttes d'huile, mais à rester en solution aqueuse en donnant des viscosités très élevées, ce qui rend les opérations difficiles voire impossibles. De plus ce polymère en formation ne se dépose autour des gouttes que quand il est devenu pauvre en OH. Alors le poids moléculaire du polymère, son degré de polymérisation et de réticulation sont tels qu'il ne se dépose pas de façon homogène en formant une couche liquide compacte. Par conséquent, il se forme une paroi poreuse.

L'intérêt de mettre un monomère organosilane, tel que du MTES ou un prépolymère insoluble dans l'eau, tel que du polysilicate d'éthyle ou autre dans la phase huileuse, est qu'il y a réaction à l'interface entre ces monomères organosilanes ou prépolymères et le polymère qui se forme dans l'eau. Ainsi, le polymère de silsesquioxane en formation se lie aux gouttes d'huile et se dépose autour d'elles beaucoup plus facilement et précocement.

Grâce à cela, l'encapsulation selon le procédé de l'invention présente un caractère interfacial qui rend les microcapsules étanches et résistantes.

Comme susmentionné, un silicate préférentiellement insoluble dans l'eau à l'état hydrolysé, tel que du polysilicate d'éthyle ou un précurseur du composé polymère de type silsesquioxane, tel que le MTMS ou le MTES, qui sont aptes à rester dans l'huile conviennent plus particulièrement car étant dans la phase huileuse, ils s'hydrolyseront beaucoup moins vite que les précurseurs présents dans l'eau. De plus, une certaine quantité de ces composés seront sous forme déjà partiellement polymérisée mais pas complètement hydrolysée (car les groupes non hydrolysés auront tendance à rester dans l'huile) et pourront par conséquent réagir avec le précurseur de silsesquioxane présent dans la phase aqueuse et l'aider à se déposer autour des gouttes d'huile.

Cet effet avantageux est également valable pour une encapsulation eau dans l'huile.

Afin, il est également possible d'ajouter à ce mélange de principes actifs lipophiles, une amine lipophile telle qu'une tributylamine ou une diméthylbenzylamine. Cette amine formera à l'interface eau/huile un complexe avec les ions fluorure de la phase aqueuse, complexe qui catalysera la réaction en accentuant son caractère interfacial.

Ce premier mélange deviendra la phase interne lipophile des microcapsules.

### Préparation de la phase continue aqueuse :

La phase continue aqueuse comprend de l'eau, un ou plusieurs acides, de préférence faibles de sorte que le pH soit inférieur à 6, et se situe de préférence entre 3 et 5. Ces acides faibles sont par exemple de l'acide acétique, de l'acide formique, de l'acide citrique.

Dans cette phase aqueuse acide, sont introduits un ou plusieurs précurseurs de composés polymères de type silsesquioxane du type R-Si(R₁R₂R₃) tels que décrit ci-dessus.

Afin de favoriser la formation de l'émulsion ou le maintien de son intégrité lors de l'encapsulation, il est possible d'introduire dans la phase continue un colloïde protecteur. Ce colloïde protecteur peut être choisi dans la liste suivante : les dérivés de cellulose tel que l'hydroxyéthylcellulose, la carboxyéthylcellulose et la méthylcellulose, les polyvinylpyrrolidones et les coplymères de polyvinylpyrrolidone, les alcools polyvinyliques plus ou moins hydrolysés ainsi que leur copolymères, les polymères d'origine naturelle comme la gélatine, la gomme de xanthane ou la gomme arabique, les alginates, pectines, amidons et dérivés, la caséine ainsi que des polymères ionisés tels que les polymères et copolymères d'acide acrylique ou méthacrylique, les polymères porteurs de groupements sulfoniques. Ces colloïdes permettent en plus d'obtenir une dispersion granulométrique de l'émulsion ou de la dispersion pas trop large et de diminuer les agglomérations au cours de la polymérisation de l'enveloppe.

### Fabrication de l'émulsion/dispersion, hydrolyse et début de polymérisation:

La phase interne lipophile est mélangée sous agitation à la phase continue aqueuse. Selon une autre variante de réalisation, il est possible d'attendre que l'hydrolyse des précurseurs du composé polymère de type silsesquioxane se fasse avant d'introduire la phase interne.

Cet ajout se fait à une température se situant entre 10°C et 50°C, de préférence entre 20°C et 40°C.

Cette opération peut être effectuée à l'aide d'agitateurs, d'homogénéisateurs, ou de turbines rotor/stator. La vitesse de rotation sert à régler la taille des microcapsules qui sera ajustée en général entre 0,1 et 100µm.

Afin de faciliter cette opération, des tensio-actifs peuvent être utilisés, mais ne sont généralement pas nécessaires. A titre d'exemple, il est possible d'utiliser : les esters d'acides gras de sorbitan ou de glycérol plus ou moins oxyéthylénés; les dérivés polyoxyéthylénés de phénols portant des chaînes grasses, les amino ou amido bétaïnes portant des chaînes grasses, les condensats d'acide gras ou d'alcools gras oxyéthylénés, les alkylarylsulfonates, les savons d'acides gras, les sulfates et sulfonates gras, les dialkylsulfosuccinates, les oxydes d'amines grasses, imidazolines grasses, amidosulfobétaïnes grasses, les émulsionnants cationiques, les mono ou diéthanolamides d'acides gras, les dispersants de type silicone tels les diméthicone copolyols , ou leurs mélanges.

La phase interne est présente dans l'émulsion ou la dispersion des microcapsules à hauteur de 35 à 40 % environ.

A ce stade, les parois des microcapsules sont liquides. Le précurseur de silsesquioxane commence à entourer la phase dispersée au fur et à mesure de son hydrolyse.

### Poursuite et accélération de la polymérisation :

Après un temps de quelques minutes à quelques heures, un ou plusieurs acides forts sont introduits. L'acide fort est de manière avantageuse de l'acide fluorhydrique seul ou en mélange avec d'autres acides forts, tels que l'acide nitrique, l'acide chlorhydrique, l'acide trifluorométhanesulfonique. La paroi durcit alors progressivement. Le pH descend aux alentours de 1 (voire 0,8) à 4, de préférence de 1,5 à 2,5.

Après une à quelques heures, la température est montée progressivement ou non jusqu'aux alentours de 65°C. La température doit être suffisamment élevée et le temps suffisamment long pour que l'alcool produit par la réaction puisse s'éliminer en grande partie par évaporation étant donné que cette réaction est partiellement réversible. Cette température peut varier de 40 à 100°C.

Pendant cette phase le nombre de groupes OH diminue dans la masse de la paroi et à la surface des microcapsules. Les microcapsules peuvent devenir alors hydrophobes et s'agglomérer malgré la présence du colloïde protecteur.

Pour remédier à cela, il est intéressant d'introduire un silane hydrophile qui va se greffer sur la surface des microcapsules pour les rendre hydrophiles de façon permanente.

Le silane convenant pour la présente invention est par exemple du type R₅-Si(R₁R₂R₃) ou R₅Si-[(CH₃)R₁R₂]
- où R₅ est un groupe hydrophile non hydrolysable tel un polyéther de glycol un groupe époxyde (apte à s'ouvrir pour donner un OH compte tenu des conditions de pH), un groupe portant une ou des fonctions acide, alcool, amine. Parmi les silanes portant une ou des fonctions amine, une famille intéressante est celle comportant une amine cationisée car elle permet de conférer aux microcapsules une charge cationique très utile dans des applications cosmétiques ou textiles par exemple pour l'affinité pour la peau ou les fibres textiles que cette charge confère ;
- et où, les groupes R₁, R₂, R₃ sont les groupes hydrolysables décrits précédemment.

Ce composé silane est introduit après solidification partielle de la paroi de façon à ce qu'il reste en surface et non dans la masse de la paroi en formation, c'est-à-dire qu'il est introduit juste avant qu'une tendance à l'agglomération (qui se traduit par un changement de viscosité) apparaisse.

Des catalyseurs métalliques ou organométalliques bien connus de l'homme de l'art peuvent être utilisés pour aider à terminer la réaction de polymérisation tels les composés renfermant de l'étain comme le dilaurate ou le diacétate de dibutylétain, l'octanoate d'étain, les sels minéraux d'étain, ainsi que les composés du platine, du zinc, du zirconium, de l'aluminium, du titane dont les titanates, par exemple.

### Remontée du pH :

Cette opération n'est pas obligatoire, mais le pH final des microcapsules obtenues étant en général situé entre 0,8 à 3,5 en fin d'encapsulation, il est difficile de les utiliser ainsi. Le pH est donc remonté vers 6,5 pour des raisons pratiques et de compatibilité avec les milieux ou les capsules sont utilisées (on peut aller de 4 à 8,5 environ). Cette opération est effectuée avec de la soude, de la potasse ou des amines.

### b) Encapsulation eau dans l'huilé

### Préparation de la phase interne aqueuse :

La phase interne hydrophile est préparée à partir de principes actifs hydrophiles, tels que des protéines ou des hydrolysats de protéine, des acides aminés (hydroxyproline, proline), des polyols tels que la glycérine, le sorbitol, le butylène glycol, le propylène glycol ou le polyéthylène glycol, de l'allantoïne, de la DHA, de la guanosine, des sucres et dérivés de sucre, des vitamines hydrosolubles telles que l'acide ascorbique (vitamine C), des hydroxyacides et leurs sels et des actifs spécifiques hydrosolubles tels que les actifs hydratants, des anti-rides, des amincissants, des nutritifs, ou des adoucissants, etc.

A ces principes actifs hydrophiles est rajoutée obligatoirement de l'eau nécessaire aux réactions d'hydrolyse et de polymérisation, et optionnellement un solvant soluble dans l'eau (par exemple du glycol, de l'alcool, leurs éthers, leurs esters, du glycérol, etc). De façon générale, tous les solvants formant avec l'eau une solution, mais qui ne sont pas solubles dans la phase continue lipophile, peuvent convenir.

Le ou les principes actifs y sont mélangés ou dissous.

Un ou plusieurs acides faibles ou forts y sont dissous, et optionnellement de l'acide fluorhydrique ou un fluorure soluble dans l'eau, de sorte à diminuer le pH. Il est possible de descendre par exemple le pH entre 1 et 4 dès l'étape d'hydrolyse du précurseur.

Il est également possible d'y introduire un composé précurseur de silsesquioxane tel que défini ci-dessus. Le MTMS ou le MTES conviennent de préférence.

Puis, le tout est agité jusqu'à ce que les précurseurs de silsesquioxane s'hydrolysent suffisamment pour devenir solubles, avant l'opération d'émulsification.

### Préparation de la phase continue lipophile :

La phase continue est une phase organique constituée d'esters, d'hydrocarbures, d'huiles, de fluide silicone, de solvants ou de leurs mélanges et de manière générale de tout milieu non miscible à l'eau et liquide dans les conditions d'encapsulation.

Il est également possible d'ajouter un précurseur de silsesquioxane. Ainsi, ce précurseur peut être présent dans une des deux phases interne ou continue, ou dans les deux à la fois.

Tout comme pour l'encapsulation eau dans l'huile, il est possible de rajouter dans la phase lipophile, un organosilicate, tel que le polysilicate d'éthyle qui est insoluble dans l'eau même à l'état hydrolysé.

### Fabrication de l'émulsion/dispersion, hydrolyse et début de polymérisation :

Comme pour l'encapsulation huile dans eau, l'ajout de la phase interne se fait sous agitation. La vitesse d'agitation est réglée pour obtenir le diamètre voulu.

La phase interne est généralement présente à hauteur de 40 à 45% du mélange des microcapsules.

Un émulsionnant tel que défini précédemment peut être ajouté, de préférence dans la phase organique.

Dans une variante, un composé précurseur de type silsesquioxane (MTES ou MTMS) ainsi qu'éventuellement du polysilicate d'éthyle peuvent être introduits à cette étape si cela n'a pas déjà été effectué.

Dans ces conditions, la polymérisation se développe et les chaînes polymères s'allongent.

### Poursuite et accélération de la polymérisation :

Après un temps de 30 mn à quelques heures, une amine lipophile, telle la tributylamine ou la diméthylbenzylamine peut être introduite dans le but de former un complexe avec un acide fort, tel que l'acide fluorhydrique de la phase aqueuse. Si cet acide n'est pas présent dès le départ dans la phase aqueuse, il est possible de faire réagir l'amine avec de l'acide fluorhydrique à part et d'introduire le mélange obtenu dans la phase organique, après la phase de début d'hydrolyse/polymérisation. Il est possible également de se passer de l'amine en introduisant avec l'acide fluorhydrique dans la phase aqueuse, un fluorure tel que du fluorure de sodium ou du fluorure de potassium.

Le polymère tridimensionnel est finalement polymérisé dans sa totalité en milieu acide. L'ajout des ions fluorure grâce à l'acide fluorhydrique ou à des composés comportant dans leur structure des ions fluorures permet de favoriser la polycondensation des groupes silanols demeurant libres au sein du mélange.

La température de départ est ambiante, mais on peut démarrer plus haut. La température finale se situe entre 40 et 80°C.

La paroi est au départ liquide et se solidifie progressivement (surtout après introduction de l'amine).

Il est possible d'introduire un silane lipophile qui va se greffer sur la surface des microcapsules pour les rendre plus lipophiles. Ce silane peut être du butyltriméthoxysilane ou du butyltriéthoxysilane. Ce silane est introduit après solidification partielle de la paroi de façon à ce qu'il reste en surface et non dans la masse de la paroi en formation. Pratiquement, on l'introduit juste avant qu'une tendance à l'agglomération, qui se traduit par un changement de viscosité, se produise.

Il est également très intéressant d'introduire dans cette phase de polymérisation un silane porteur de fonctions amine dont l'une au moins est cationisée. Ceci conduit en effet à des microcapsules portant une charge cationique. Ce type de modification de surface améliore beaucoup les possibilités d'émulsification du mélange organique de microcapsules dans l'eau, ce qui est intéressant dans de nombreuses applications dont le textile. Ici encore, il est possible d'ajouter un catalyseur métallique tel que précédemment décrit pour accélérer les réactions.

### Remontée du pH :

Cette opération n'est pas non plus obligatoire, mais il est possible de remonter le pH de la phase interne en introduisant une base (amine organique principalement) dans la phase organique, de manière à obtenir un pH entre 5,5 et 8,5.

Ensuite, les microcapsules comprenant une émulsion ou une dispersion eau dans huile ou huile dans l'eau peuvent ensuite être séchées dans une tour d'atomisation, ou sur lit fluidisé, ou par cryoséchage ou tous autres moyens équivalents.

Pour obtenir des microcapsules étanches, il faut que la paroi soit compacte et non poreuse. Comme décrit ci-dessus, ceci peut être obtenu en polymérisant la paroi très progressivement de façon à ce qu'elle reste liquide le plus longtemps possible et ne se solidifie qu'en fin d'opération par augmentation du poids moléculaire et de réticulations.

Pour mieux faire comprendre l'objet de l'invention, des modes de réalisation vont être décrits, à titre d'exemples purement illustratifs et non limitatifs de la portée de l'invention.

### EXEMPLES :

### Exemple 1: Microcapsules de polyméthylsilsesquioxane contenant un actif cosmétique

Dans un bécher de 500 cm³ maintenu à 40°C on introduit sous agitation 70 g d'eau du robinet, 1,4 g d'acide citrique à 40 %, 16,0 g d'un copolymère pyrrolidone/vinylacétate (Collacral VAL de BASF).

La vitesse d'agitation est augmentée, puis un mélange de 86 g d'huile de karité lipex 205 (vendu par Unipex) et de 0,72 g de tributylamine est introduit pour être émulsionné puis 40 g de MTES (Dynasylan MTES de Degussa). Au bout de 40 mn à 40°C le mélange suivant est ajouté : 12 g de PEGg 14M à 6% dans l'eau (300 000 à 400 000 de poids moléculaire) de la société Biosynthis, 3,0 g d'acide trifluorométhane sulfonique à 20 % dans l'eau et 9,2 g d'acide fluorhydrique à 20 % dans l'eau.

La température est maintenue à 40°C pendant 2h, et l'agitation est réglée pour obtenir un diamètre de microcapsules de 20µm.

Puis, 4,0 g de glycidoxypropylméthyldiéthoxysilane (Wetlink 78 de Momentive) sont introduits pour conserver l'hydrophilie des microcapsules. La température est alors montée à 65°C et maintenue 12h, des ajouts d'eau étant effectués pour maintenir le niveau qui baisse par suite de l'évaporation (perte d'alcool et d'eau).

L'émulsion est lentement refroidie à 25°C. Le pH est ensuite remonté lentement à 6 avec une solution de lessive de soude à 30 %.

### Exemple 2: Microcapsules de polyméthylsilsesquioxane contenant un actif cosmétique

Dans un bécher de 300 cm³ maintenu à 40°C on introduit sous agitation : 35 g d'eau du robinet, 2,5 g d'acide citrique à 40 %, 1,5 g d'acide trifluorométhanesulfonique à 20 %, 1,0 g d'acide chlorhydrique à 20 %, 6,0 g d'un copolymère pyrrolidone/vinylacétate (Collacral VAL de BASF), 15,0 g de MTES, 0,5 g de 3-aminopropylméthyldiéthoxysilane (Dynasylan 1505 de Degussa).

La vitesse d'agitation est augmentée, puis un mélange de : 43 g de squalène d'huile d'olive, 5g de MTES, 0,36 g de tributylamine, préalablement porté à 50°C et homogénéisé, est introduit pour être émulsionné. L'agitation est réglée pour obtenir un diamètre de 15µm.

Au bout de 15 mn le mélange suivant est ajouté: 4 g de solution de PEGg 14M à 6% dans l'eau (300 000 à 400 000 de poids moléculaire) de la société Biosynthis et 4,6 g d'acide fluorhydrique à 20 % dans l'eau.

La température est maintenue à 40°C pendant 1h30. 2,0 g de glycidoxypropylméthyldiéthoxysilane (Wetlink 78 de Momentive) sont introduits pour conserver l'hydrophilie des microcapsules.

La température est alors montée à 65°C et maintenue 12h, des ajouts d'eau étant effectués pour maintenir le niveau qui baisse par suite de l'évaporation (perte d'alcool et d'eau).

L'émulsion est lentement refroidie à 25°C. Le pH est monté lentement à 6.0 avec une solution de lessive de soude à 30 %

### Exemple 3: Microcapsules de polyméthylsilsesquioxane copolymère contenant un parfum

Dans un réacteur de 800 cm³ maintenu à 25°C, on introduit sous agitation : 168 g d'eau du robinet, 1,4 g d'acide acétique à 65%, 77,0 g de MTMS (Dynasylan MTMS de Degussa).

Le mélange est agité pendant 20 mn à 25°C

On ajoute alors le mélange : 14 g d'eau du robinet, 7 g d'acide trifluorométhane sulfonique à 20 % dans l'eau, et 21 g d'acide fluorhydrique à 20 % dans l'eau.

Sont ajoutés alors sous plus forte agitation pour fabriquer l'émulsion :
1) le mélange de 196 gr de parfum Rose freesia 07 006 02(expression parfumées), 17,5 g de tripropylèneglycol-n-butyl- éther (Dowanol TPnB de Dow), 56 g de polysilicate d'éthyle (Dynasil 40 de Degussa) et 2 g de tributylamine.
2) 17,5g d'un copolymère pyrrolidone/vinylacétate (Collacral VAL de BASF)

La température est maintenue pendant 1h30 à 25°C, puis 2h à 40°C, puis 30 mn à 75°C. Pendant ce temps, l'agitation est réglée pour obtenir un diamètre de 6µm.

12,0 g de Wetlink 78 (de Momentive) sont introduits pour conserver l'hydrophilie des microcapsules.

La température est maintenue à 75°C pendant 3h30, des ajouts d'eau étant effectués pour maintenir le niveau qui baisse par suite de l'évaporation (perte d'alcool et d'eau).

L'émulsion est lentement refroidie à 25°C. 16h plus tard le pH est remonté lentement à 6,5 avec une solution de lessive de soude à 30 %.

### Exemple 4: Microcapsules de polyméthylsilsesquioxane copolymère contenant un matériau à changement de phase (PCM)

Dans un réacteur de 2,5 litres maintenu à 35°C est introduit sous agitation : 440 g d'eau du robinet, 5,5 g d'acide acétique à 65 % et 357,5 g de MTMS (Dynasylan MTMS de Degussa).

Le mélange est agité pendant 20 mn à 35°C

On ajoute alors successivement : 412 g d'une solution à 8% de PVA carboxylé dans de l'eau du robinet (Poval KL318 de Kuraray). Puis, on introduit lentement le mélange constitué de : 770 g de l'actif RT31 (paraffine fondant à 31°C de la société Rubitherm) préalablement mélangé à 192 g de polysilicate d'éthyle (dynasil 40 de Degussa) et porté à 35°C, et on émulsionne.

Puis le mélange de : 13,75 g d'acide trifluorométhanesulfonique à 20 % dans l'eau, 35,75 g d'acide fluorhydrique à 20 % dans l'eau et 55 g d'eau du robinet, est ajouté.

La vitesse de l'agitateur est réglée pour obtenir un diamètre de 6µ et le tout est maintenu pendant 3h à 35°C.

On ajoute alors 6,9 g de tributylamine et on maintient pendant 1h à 35°C. Puis on chauffe pendant 1h30 à 45°C et ensuite pendant 3h à 75°C.

On laisse refroidir et le lendemain on remonte le pH à 6.0 avec de la lessive de soude à 30 %.

### Exemple 5: Microcapsules de polymethylsilsesquioxane copolymère contenant un actif aqueux

Dans une cuve de 3 litres à double enveloppe est introduit sous agitation : 220 g d'isononanoate d'isononyle, 879 g de cyclopentasiloxane, 161 g de polysilicate d'éthyle (Dynasil 40 de Degussa), 4,4 g de cétyl diméthicone copolyol (Abil EM 90 de goldschmidt).

Une fois cette phase continue homogène, on vient y disperser sous forte agitation la phase aqueuse constituée du mélange : 988 g d'une solution de sulfate d'aluminium à 30 %, 29,3 g d'acide fluorhydrique à 20% dans l'eau et 11,7g de AMP (2-amino-2-méthyl-1-propanol) à 50 % dans l'eau.

Puis, on introduit dans l'émulsion 190 g de MTMS (Dynasylan MTMS de Degussa).

On maintient à la température ambiante pendant 1 heure à 25°C, puis 2h à 40°C, puis 2h à 60°C. On règle l'agitation pour obtenir un diamètre de 20µm.

On introduit alors 7,3 g de triéthanolamine, puis 3,5 g de diacétate de dibutylétain.

On maintient pendant 4h à 60°C puis on laisse refroidir.

### Exemple 6: Microcapsules de polyméthylsilsesquioxane copolymère contenant un actif aqueux

Dans un bécher de 350 ml plongé dans un bain marie à 20°C, est introduit sous agitation : 40 g d'isononanoate d'isononyle, 40 g de cocoate de 2-éthylhexyle, 10 g de cyclopentasiloxane, 9,6 g de polysilicate d'éthyle (Dynasil 40 de Degussa), 0,2 g de triéhylamine et 1 g de cétyl diméthicone copolyol (abil EM 90 de goldschmidt).

Une fois cette phase continue homogène, on vient y disperser sous forte agitation la phase aqueuse préalablement homogénéisée : 74 g de « Fleur de bach » (extrait aqueux), 3,0 g d'acide fluorhydrique à 20 % dans l'eau et 0,2 g de triéthylamine.

Puis on introduit dans l'émulsion, 20 g de MTMS (Dynasylan MTMS de Degussa)
La température est maintenue à 20°C pendant 2 heures, puis à 40°C pendant 2h. L'agitation est réglée pour obtenir un diamètre de 8µm.

On introduit alors 2,0 g d'un aminosilane cationique (Dynasylan 1172 de Degussa). Puis on maintient le mélange pendant 4h à 40°C. Le mélange organique de microcapsules obtenu est facilement émulsionnable dans l'eau du fait des charges cationiques fixées sur les microcapsules.

### Exemple 7: Microcapsules de polyméthylsilsesquioxane copolymère contenant un actif aqueux

Dans un bécher de 250 ml à double enveloppe est introduit sous agitation :
12,30 g d'isononanoate d'isononyle, 49,1 g de cyclopentasiloxane, 9,9 g de polysilicate d'éthyle (Dynasil 40 de Degussa), 0,3 g de cétyl diméthicone copolyol (Abil EM 90 de goldschmidt).

Dans un bécher de 100 ml, on mélange 60,7 ml de solution de glutataldehyde à 50 % dans l'eau à 1,8 g de solution d'acide fluorhydrique à 20%. On disperse dans ce mélange 1,4 g de MTES (Dynasylan MTES de Degussa) à la température ambiante.

Au bout de 15mn la phase aqueuse devient transparente. On l'émulsionne alors sous forte agitation dans la phase organique précédente, puis on introduit dans l'émulsion 12,5 g de MTMS (Dynasylan MTMS de Degussa).

On maintient à la température ambiante pendant 1 heure, pendant que l'on règle l'agitation de façon à obtenir un diamètre de 8µm, puis on introduit 0,5 g de tributylamine.

On chauffe alors pendant 2h à 40°C, puis pendant 1h à 60°C.

On introduit ensuite 0,1 g de dibutylétaindiacétate.

On maintient pendant 2h à 60°C pour terminer la réaction, puis on laisse refroidir.

Les exemples 1 à 7 permettent d'obtenir des microcapsules à réservoir dont la paroi est formée de silsesquioxane, qui sont étanches et résistantes.

## Revendications

1. Procédé de fabrication de microcapsules à réservoir comprenant un noyau contenant au moins un principe actif, ledit noyau étant entouré par une enveloppe polymère formée de 50 à 100 % en poids d'un composé de silsesquioxane par rapport au poids total de ladite enveloppe, ledit procédé comprenant les étapes consistant à :
(i) disperser au moins un principe actif lipophile ou hydrophile dans une phase continue respectivement aqueuse ou organique, de manière à former une émulsion ou une dispersion huile dans l'eau ou eau dans l'huile, respectivement,
(ii) hydrolyser un précurseur du composé polymère de type silsesquioxane de formule R- SiO_{3/2} où R est :
- un radical alkyle à 1 à 20 atomes de carbone, substitué ou non tel que des radicaux méthyle, éthyle, n-propyle, isopropyle, 1-n-butyle, 2-n-butyle, isobutyle, tert-butyle, n-pentyle, isopentyle, néopentyle, tert-pentyle, hexyle, heptyle, octyle, 2,2,4-triméthylpentyle, nonyle, décyle, dodécyle, octadécyle, cycloalkyle, aryle, alkaryle, et aralkyle,
- un radical alkyle oxygéné, tel les méthoxyéthyle et éthoxyéthyle,
- un radical halogéné tel que le chloropropyle, 3,3,3-trifluoro-n- propyle, 2,2,2,2,2',2',2'-hexafluoroisopropyle, heptafluoroisopropyle, o-, m- et p-chlorophényle,
- ou un radical insaturé tel que vinyle, 5-hexényle, 2,4-divinylcyclohexyléthyle, 2-propényle, allyle, 3-butényle, 4-pentényle, éthynyle, propargyle et 2-propynyle.
et le polymériser *in situ* dans ou en contact de la phase aqueuse de la dispersion ou de l'émulsion huile dans l'eau ou eau dans l'huile, de manière à former un homopolymère ou copolymère silsesquioxane
caractérisé en ce (iii) qu'on introduit dans la phase organique des microcapsules en début de réaction d'hydrolyse et/ou de polymérisation, un composé choisi parmi :
- un silicate préférentiellement insoluble dans l'eau à l'état hydrolysé, tel que du polysilicate d'éthyle,
- un précurseur du composé polymère de type silsesquioxane,
- ou leurs mélanges,
et en ce que l'étape de polymérisation s'effectue en milieu acide,
de sorte à conférer à la polymérisation ou à l'encapsulation un caractère interfacial favorable à l'étanchéité des microcapsules.

2. Procédé de fabrication de microcapsules à réservoir selon la revendication 1, dans lequel le pH lors de la polymérisation est inférieur à 6.

3. Procédé de fabrication de microcapsules à réservoir selon l'une des revendications 1 ou 2, dans lequel le pH est situé entre 3 et 5 lors de l'hydrolyse et lors du début de la polymérisation, puis de 1 à 4, de préférence de 1,5 à 2, 5 jusqu'à la fin de la polymérisation.

4. Procédé de fabrication de microcapsules à réservoir selon l'une des revendications 1 ou 2, dans lequel le pH se situe entre 1 et 4 dès l'étape d'hydrolyse.

5. Procédé de fabrication de microcapsules à réservoir selon l'une des revendications précédentes, dans lequel des ions fluorures ou un ou plusieurs composés comportant dans leur structure des ions fluorures sont présents dans le milieu au cours de la polymérisation.

6. Procédé de fabrication de microcapsules à réservoir selon l'une des revendications précédentes, dans lequel les ions fluorures sont utilisés en présence d'un composé portant une fonction amine.

7. Procédé de fabrication de microcapsules à réservoir selon l'une des revendications précédentes, dans lequel le pH en fin de réaction de polymérisation est remonté entre 5,5 et 8,5, de préférence entre 6 et 7.

8. Procédé de fabrication de microcapsules à réservoir selon l'une des revendications précédentes, dans lequel, dans le cas d'une émulsion huile dans eau, un ou des silanes porteurs de groupes hydrophiles sont introduits après solidification au moins partielle de la paroi des microcapsules.

9. Procédé de fabrication de microcapsules à réservoir selon l'une des revendications précédentes, dans lequel dans le cas d'une émulsion eau dans l'huile, un ou des silanes porteurs de groupes lipophiles sont introduits après solidification au moins partielle de la paroi des microcapsules.

10. Procédé de fabrication de microcapsules à réservoir selon l'une des revendications 8 ou 9, dans lequel au moins un silane porte des charges cationiques.

11. Procédé de fabrication de microcapsules à réservoir selon l'une des revendications 1 à 10, dans lequel la température se situe entre 10°C et 50°C lors de l'étape de dispersion ou d'hydrolyse, puis de 40°C à 90°C lors de l'étape de polymérisation.

12. Procédé de fabrication de microcapsules à réservoir selon l'une des revendications 1 à 11, dans lequel le précurseur du composé polymère de type silsesquioxane est du type R-Si(R₁R₂R₃),
où R est tel que défini précédemment,
où R₁,R₂,R₃ désignent chacun indépendamment un groupe : acétoxy, amino, acide amide, oximino, chlore, un groupe OR₄ où R₄ est :
- un radical alkyle à 1 à 3 atomes de carbone, substitué ou non tels par exemple par des radicaux méthyle, éthyle, n-propyle, isopropyle,
- un radical alkyle oxygéné, tel que les méthoxyéthyle et éthoxyéthyle,
- ou un radical insaturé tel que vinyle, allyle.

13. Procédé de fabrication de mircocapsules à réservoir selon la revendication 12, dans lequel le précurseur du composé polymère de type silsesquioxane est le méthyltriméthoxysilane (MTMS), le méthyltriéthoxysilane (MTES), le méthyltrichlorosilane ou leurs mélanges.

14. Procédé de fabrication de microcapsules à réservoir selon l'une quelconque des revendications précédentes, **caractérisé en ce que** le principe actif est un filtre UV.

## Patentansprüche

1. Herstellungsverfahren von Mikrokapseln mit Reservoir, umfassend einen Kern, enthaltend mindestens einen Wirkstoff, wobei der Kern von einer Polymerhülle umgeben ist, die von 50 bis 100 Gew.-% von einer Silsesquioxanverbindung in Bezug zum Gesamtgewicht der Hülle gebildet ist, wobei das Verfahren die Schritte umfasst, die darin bestehen:
(i) Dispergieren mindestens eines lipophilen oder hydrophilen Wirkstoffs in einer kontinuierlichen jeweils wässrigen oder organischen Phase, um jeweils eine Emulsion oder eine Dispersion Öl-in-Wasser oder Wasser-in-Öl zu bilden,
(ii) Hydrolysieren eines Vorläufers der Polymerverbindung vom Typ Silsesquioxan der Formel R-SiO_{3/2}, wobei R ist:
- ein Alkylradikal mit 1 bis 20 Kohlenstoffatomen, substituier oder nicht, wie Methyl-, Ethyl-, n-Propyl-, Isopropyl-, 1-n-Butyl-, 2-n-Butyl-, Isobutyl-, tert-Butyl-, n-Pentyl-, Isopentyl-, Neopentyl-, tert-Pentyl-, Hexyl-, Heptyl-, Octyl-, 2,2,4-Trimethylpentyl-, Nonyl-, Decyl-, Dodecyl-, Octadecyl-, Cycloalkyl-, Aryl-, Alkaryl- und Aralkylradikale,
- ein oxygeniertes Alkylradikal wie das Methoxyethyl und Ethoxyethyl,
- ein Halogenradikal wie das Chloropropyl, 3,3,3-Trifluor-n-propyl, 2,2,2,2',2',2'-Hexafluorisopropyl, Heptafluorisopropyl, o-, m- und p-Chlorphenyl,
- oder ein ungesättigtes Radikal wie Vinyl, 5-Hexenyl, 2,4-Divinylcyclohexylethyl, 2-Propenyl, Allyl, 3-Butenyl, 4-Pentenyl, Ethynyl, Propargyl und 2-Propynyl,
und dessen Polymerisierung *in situ* in oder im Kontakt mit der wässrigen Phase der Dispersion oder der Emulsion Öl-in-Wasser oder Wasser-in-Öl, um ein Silsesquioxan-Homopolymer oder -Copolymer zu bilden,
**dadurch gekennzeichnet, dass** (iii) bei der organischen Phase der Mikrokapseln zu Beginn der Hydrolyse- und/oder Polymerisationsreaktion eine Verbindung eingeführt wird, ausgewählt aus:
- einem vorzugsweise in Wasser in hydrolysiertem Zustand unlöslichen Silikat wie Ethylpolysilikat,
- einem Vorläufer der Polymerverbindung vom Typ Silsesquioxan,
- oder deren Gemischen,
und dass der Polymerisationsschritt im sauren Milieu stattfindet,
so dass der Polymerisation oder der Einkapselung ein Interface-Charakter verliehen wird, der für die Dichtigkeit der Mikrokapseln günstig ist.

2. Herstellungsverfahren von Mikrokapseln mit Reservoir nach Anspruch 1, wobei der pH bei der Polymerisation unter 6 ist.

3. Herstellungsverfahren von Mikrokapseln mit Reservoir nach einem der Ansprüche 1 oder 2, wobei sich der pH zwischen 3 und 5 bei der Hydrolyse und am Beginn der Polymerisation, dann von 1 bis 4, vorzugsweise von 1,5 bis 2,5 bis zum Ende der Polymerisation, befindet.

4. Herstellungsverfahren von Mikrokapseln mit Reservoir nach einem der Ansprüche 1 oder 2, wobei sich der pH zwischen 1 und 4 ab dem Hydrolyseschritt befindet.

5. Herstellungsverfahren von Mikrokapseln mit Reservoir nach einem der vorangehenden Ansprüche, wobei Fluoridionen oder eine oder mehrere Verbindungen, die in ihrer Struktur Fluoridionen aufweisen, in dem Milieu während der Polymerisation vorhanden sind.

6. Herstellungsverfahren von Mikrokapseln mit Reservoir nach einem der vorangehenden Ansprüche, wobei die Fluoridionen bei Anwesenheit einer Verbindung verwendet werden, die eine Aminfunktion trägt.

7. Herstellungsverfahren von Mikrokapseln mit Reservoir nach einem der vorangehenden Ansprüche, wobei sich der pH am Ende der Polymerisationsreaktion zwischen 5,5 und 8,5, vorzugsweise zwischen 6 und 7, befindet.

8. Herstellungsverfahren von Mikrokapseln mit Reservoir nach einem der vorangehenden Ansprüche, wobei bei einer Öl-in-Wasser-Emulsion ein oder Silane als Träger hydrophiler Gruppen nach mindestens teilweiser Verfestigung der Wand der Mikrokapseln eingeführt werden.

9. Herstellungsverfahren von Mikrokapseln mit Reservoir nach einem der vorangehenden Ansprüche, wobei bei einer Wasser-in-Öl-Emulsion ein oder Silane als Träger lipophiler Gruppen nach mindestens teilweiser Verfestigung der Wand der Mikrokapseln eingeführt werden.

10. Herstellungsverfahren von Mikrokapseln mit Reservoir nach einem der Ansprüche 8 oder 9, wobei mindestens ein Silan kationische Ladungen trägt.

11. Herstellungsverfahren von Mikrokapseln mit Reservoir nach einem der Ansprüche 1 bis 10, wobei sich die Temperatur zwischen 10 °C und 50 °C beim Dispersions- oder Hydrolyseschritt, danach von 40 °C bis 90 °C beim Polymerisationsschritt befindet.

12. Herstellungsverfahren von Mikrokapseln mit Reservoir nach einem der Ansprüche 1 bis 11, wobei der Vorläufer der Polymerverbindung vom Typ Silsesquioxan vom Typ R-Si(R₁R₂R₃) ist,
wobei R wie zuvor definiert ist,
wobei R₁, R₂, R₃ bezeichnen, jeweils unabhängig, eine Gruppe: Acetoxy, Amin, Amidsäure, Oximin, Chlor, eine Gruppe OR₄, wobei R₄ ist:
- ein Alkylradikal mit 1 bis 3 Kohlenstoffatomen, substituiert oder nicht, wie beispielsweise von Methyl-, Ethyl-, n-Propyl-, Isopropylradikalen,
- ein oxygeniertes Alkylradikal wie das Methoxyethyl und Ethoxyethyl,
- oder ein ungesättigtes Radikal wie Vinyl, Allyl.

13. Herstellungsverfahren von Mikrokapseln mit Reservoir nach Anspruch 12, wobei der Vorläufer der Polymerverbindung vom Typ Silsesquioxan das Methyltrimethoxysilan (MTMS), das Methyltriethoxysilan (MTES), das Methyltrichlorosilan oder deren Gemische ist.

14. Herstellungsverfahren von Mikrokapseln mit Reservoir nach einem der vorangehenden Ansprüche, **dadurch gekennzeichnet, dass** der Wirkstoff ein UV-Filter ist.

## Claims

1. Method for producing core-shell microcapsules comprising a core containing at least one active ingredient, said core being surrounded by a polymer shell formed of 50 to 100 % by weight of a silsesquioxane compound relative to the total weight of said shell, said method comprising the steps of:
i) dispersing at least one lipophilic or hydrophilic active ingredient in a continuous aqueous or organic phase respectively, to form an oil-in-water or water-in-oil emulsion or dispersion respectively;
ii) hydrolysing a precursor of the polymer compound of silsesquioxane type of formula R-SiO_{3/2} where R is:
- an alkyl radical having 1 to 20 carbon atoms, substituted or unsubstituted such as methyl, ethyl, n-propyl, isopropyl, 1-n-butyl, 2-n-butyl, isobutyl, tert-butyl, n-pentyl, isopentyl, neopentyl, tert-pentyl, hexyl, heptyl, octyl, 2,2,4-trimethylpentyl, nonyl, decyl, dodecyl, octadecyl, cycloalkyl, aryl, alkaryl and aralkyl radicals;
- an oxygenated alkyl radical such as methoxyethyl and ethoxyethyl,
- a halogenated radical such as chloropropyl, 3,3,3-trifluoro-n-propyl, 2,2,2,2',2',2'-hexafluoroisopropyl, heptafluoroisopropyl, o-, m- and p-chlorophenyl;
- or an unsaturated radical such as vinyl, 5-hexenyl, 2,4-divinylcyclohexylethyl, 2-propenyl, allyl, 3-butenyl, 4-pentenyl, ethynyl, propargyl and 2-propynyl,
and *in situ* polymerisation thereof in or in contact with the aqueous phase of the oil-in-water or water-in-oil dispersion or emulsion, to form a silsesquioxane homopolymer or copolymer;
**characterized in that** (iii) adding to the organic phase of the microcapsules, at the start of the hydrolysis and/or polymerisation reaction, of a compound selected from among:
- a silicate, preferably water-insoluble in the hydrolysed state, such as ethyl polysilicate,
- a precursor of the polymer compound of silsesquioxane type,
- or mixtures thereof
and **in that** the polymerisation step is conducted in an acid medium,
so as to impart an interfacial nature to polymerisation or encapsulation, to promote imperviousness of the microcapsules.

2. The method for producing core-shell microcapsules according to claim 1, wherein the pH at polymerisation is lower than 6.

3. The method for producing core-shell microcapsules according to one of claims 1 or 2, wherein the pH lies between 3 and 5 during hydrolysis and at the start of polymerisation, then 1 to 4, preferably 1.5 to 2.5 up until the end of polymerisation.

4. The method for producing core-shell microcapsules according to one of claims 1 or 2, wherein the pH lies between 1 and 4 starting at the hydrolysis step.

5. The method for producing core-shell microcapsules according to one of the preceding claims wherein fluoride ions, or one or more compounds comprising fluoride ions within their structure, are contained in the medium during polymerisation.

6. The method for producing core-shell microcapsules according to one of the preceding claims, wherein the fluoride ions are used in the presence of a compound carrying an amine function.

7. The method for producing core-shell microcapsules according to one of the preceding claims, wherein the pH at the end of the polymerisation reaction is brought to between 5.5 and 8.5, preferably between 6 and 7.

8. The method for producing core-shell microcapsules according to one of the preceding claims wherein, for an oil-in-water emulsion, one or more silanes carrying hydrophilic groups are added after at least partial solidification of the wall of the microcapsules.

9. The method for producing core-shell microcapsules according to one of the preceding claims, wherein, for a water-in-oil emulsion, one or more silanes carrying lipophilic groups are added after at least partial solidification of the wall of the microcapsules.

10. The method for producing core-shell microcapsules according to one of claims 8 or 9, wherein at least one silane carries cationic charges.

11. The method for producing core-shell microcapsules according to one of claims 1 to 10, wherein the temperature is in the region of between 10°C and 50°C at the dispersion or hydrolysis step, then between 40°C and 90°C at the polymerisation step.

12. The method for producing core-shell microcapsules according to one of claims 1 to 11, wherein the precursor of the polymer compound of silsesquioxane type is of R-Si(R₁R₂R₃) type, where R is such as previously defined,
where R₁,R₂,R₃ are each independently a group from among: acetoxy, amino, amide acid, oximino, chlorine, an OR₄ group where R₄ is:
- an alkyl radical having 1 to 3 carbon atoms, substituted or unsubstituted, for example by methyl, ethyl, n-propyl, isopropyl radicals:
- an oxygenated alkyl radical such as methoxyethyl and ethoxyethyl;
- or an unsaturated radical such as vinyl, allyl.

13. The method for producing core-shell microcapsules according to claim 12, wherein the precursor of the polymer compound of silsesquioxane type is methyltrimethoxysilane (MTMS), methlyltriethoxysilane (MTES), methyltrichlorosilane or the mixtures thereof.

14. The method for producing core-shell microcapsules according to any of the preceding claims, **characterized in that** the active ingredient is a UV filter.
